(19) Europäisches Patentamt
European Patent Office
Office européen des brevets

(11) Veröffentlichungsnummer: **0 464 252 B1**

(12) **EUROPÄISCHE PATENTSCHRIFT**

(45) Veröffentlichungstag der Patentschrift: **19.10.94**

(51) Int. Cl.5: **A61N 1/365**

(21) Anmeldenummer: **90112955.1**

(22) Anmeldetag: **06.07.90**

(54) **Anordnung zur Gewebestimulation.**

(43) Veröffentlichungstag der Anmeldung:
**08.01.92 Patentblatt 92/02**

(45) Bekanntmachung des Hinweises auf die Patenterteilung:
**19.10.94 Patentblatt 94/42**

(84) Benannte Vertragsstaaten:
**DE FR GB IT NL SE**

(56) Entgegenhaltungen:
**US-A- 4 913 146**

(73) Patentinhaber: **Siemens Elema AB**
**Röntgenvägen 2**
**S-171 95 Solna 1 (SE)**

(84) Benannte Vertragsstaaten:
**SE**

(73) Patentinhaber: **SIEMENS AKTIENGESELL-SCHAFT**
**Wittelsbacherplatz 2**
**D-80333 München (DE)**

(84) Benannte Vertragsstaaten:
**DE FR GB IT NL**

(72) Erfinder: **Ekwall, Christer**
**Äsvägen 4**
**S-163 57 Spänga (SE)**

EP 0 464 252 B1

Rank Xerox (UK) Business Services
(3.10/3.09/3.3.3)

## Beschreibung

Die Erfindung betrifft eine Anordnung zur Gewebestimulation bei einem Lebewesen mit einem Stimulationsimpulsgenerator zur Erzeugung von Stimulationsimpulsen, mit einer an dem Stimulationsimpulsgenerator angeschlossenen Elektrodenanordnung zur Abgabe der Stimulationsimpulse an das Gewebe, mit einer das elektrische Potential im Gewebe erfassenden Detektoreinrichtung, die eine Subtrahiereinrichtung zur Erzeugung eines Differenzsignals aus dem erfaßten elektrischen Potential und einem durch die Stimulation im Gewebe hervorgerufene Polarisationserscheinungen repräsentierenden Signalverlauf enthält und eine der Subtrahiereinrichtung nachgeordnete Auswerteeinrichtung zur Auswertung des Differenzsignals auf das Vorhandensein oder Fehlen einer Reaktion des Gewebes als Folge der Stimulation aufweist, und mit einer Steuereinrichtung zur Steuerung des Stimulationimpulsgenerators in Abhängigkeit von dem Ergebnis der Auswertung des Differenzsignales.

Bei einer derartigen, aus der US-A-4 537 201 bekannten Anordnung handelt es sich um einen Herzschrittmacher, mit dem das Herz eines Patienten mit Hilfe von elektrischen Stimulationsimpulsen angeregt wird, die von einem Stimulationsimpulsgenerator erzeugt und über eine Elektrodenanordnung an das Herz abgegeben werden. Dabei erfolgt eine Kontraktion als Reaktion des Herzens auf die Stimulation nur dann, wenn die Stimulationsenergie eine bestimmte, sich aus der Stimulationsempfindlichkeit des Herzgewebes ergebende Reizschwelle überschreitet. Um die Stimulationsenergie an die veränderbare Stimulationsempfindlichkeit anpassen zu können und auf diese Weise den Energieverbrauch bei dem bekannten Herzschrittmacher gering halten zu können, wird mittels einer Detektoreinrichtung das elektrische Potential in dem zu stimulierenden Herzgewebe erfaßt und nach jeder Stimulation daraufhin ausgewertet, ob eine Stimulationsantwort, d.h. eine Reaktion des Herzgewebes als Folge der Stimulation vorliegt oder nicht. Jede Stimulation verursacht jedoch in dem Herzgewebe Polarisationserscheinungen mit einer vielfach höheren Amplitude als die zu detektierende potentielle Stimulationsantwort. Um eine Identifizierung der Stimulationsantwort in dem von der Detektoreinrichtung gemessenen elektrischen Potentialverlauf zu ermöglichen, enthält die Detektoreinrichtung des bekannten Herzschrittmachers einen Funktionsgenerator, der nach jedem Stimulationsimpuls einen die Polarisationserscheinungen durch eine logarithmische Zeitfunktion beschreibenden Signalverlauf erzeugt; dieser Signalverlauf wird in einer Subtrahiereinrichtung von dem Verlauf des im Herzgewebe gemessenen elektrischen Potentials subtrahiert und das so erhaltene Differenzsignal in einer Auswerteeinrichtung auf das Vorhandensein oder Fehlen einer Stimulationsantwort ausgewertet.

Bei einer alternativen Ausführung des bekannten Herzschrittmachers wird der von der Detektoreinrichtung im Gewebe erfaßte elektrische Potentialverlauf in einem antilogarithmischen Verstärker linearisiert, wobei anschließend in einer Auswerteeinrichtung unlineare Abweichungen von dem linearisierten Signalverlauf als Stimulationsantwort des Herzens detektiert werden.

Bei den beiden Ausführungsformen des bekannten Herzschrittmachers beruht die Kompensation der Polarisationserscheinungen in dem erfaßten Potentialverlauf auf der Annahme, daß sich der Verlauf der Polarisationserscheinungen hinreichend genau durch eine bestimmte mathematische Formel beschreiben läßt. Patientenindividuelle und sonstige systematische und unsystematische Abweichungen des tatsächlichen Verlaufs der Polarisationserscheinungen von dem angenommenen Polarisationsverlauf bleiben dabei jedoch unberücksichtigt.

Bei einem anderen, aus der EP-A-0 017 848 bekannten Herzschrittmacher ist ein sogenannter Polarisations-Kompensationsschaltkreis vorgesehen, in dem von dem gemessenen elektrischen Potentialverlauf des Herzens die Polarisationskomponente subtrahiert wird; das so erhaltene Differenzsignal wird auf das Vorhandensein von QRS- und T-Wellen ausgewertet. Auf welche Weise die zu subtrahierende Polarisationskomponente erhalten wird, geht jedoch aus der EP-A-0 017 848 nicht hervor.

Der Erfindung liegt die Aufgabe zu Grunde, bei der Erfassung des elektrischen Potentials in dem zu stimulierenden Gewebe die von der Stimulation hervorgerufenen Polarisationserscheinungen so weit wie möglich zu kompensieren, so daß eine sichere Detektion der Reaktion des Gewebes auf die Stimulation möglich ist.

Gemäß der Erfindung wird die Aufgabe dadurch gelöst, daß bei der Anordnung der eingangs angegebenen Art nach einer Stimulation der von der Detektoreinrichtung erfaßte zeitliche Verlauf des elektrischen Gewebepotentials in einem ersten Speicher zwischengespeichert wird, daß der die Polarisationserscheinungen repräsentierende Signalverlauf in einem weiteren Speicher abgespeichert ist und daß bei einem von der Auswerteeinrichtung detektierten Fehlen einer Reaktion des Gewebes auf die Stimulation der zwischengespeicherte zeitliche Verlauf des elektrischen Gewebepotentials zur Aktualisierung des Signalverlaufs in dem weiteren Speicher herangezogen wird.

Der wesentliche Vorteil der Erfindung besteht darin, daß zur Kompensation der in dem erfaßten elektrischen Potentialverlauf enthaltenen Polarisationskomponente anstelle eines angenommenen Signalverlaufs ein aktueller gemessener Verlauf der Polarisationserscheinungen herangezogen wird, so daß ein bestmöglicher Kompensationseffekt erzielt wird. Der Verlauf der Polarisationserscheinung läßt sich in einfacher Weise dadurch erfassen, daß das Gewebe mit einer unter der Reizschwelle liegenden Stimulationsenergie stimuliert wird, so daß der anschließend von der Detektoreinrichtung erfaßte Verlauf des elektrischen Potentials im Gewebe den durch den Stimulationsversuch hervorgerufenen Polarisationserscheinungen entspricht, ohne daß diese von einer Stimulationsantwort des Gewebes überlagert sind. Durch regelmäßige Aktualisierung des die Polarisationserscheinungen repräsentierenden Signalverlaufs in dem ersten Speicher wird bei der Erfassung des elektrischen Potentialverlaufs zum Zwecke der Detektion einer Stimulationsantwort stets eine optimale Kompensation der in dem erfaßten Potentialverlauf enthaltenen Polarisationskomponente erreicht.

Die Aktualisierung des abgespeicherten Signalverlaufs läßt sich in technisch einfachster Weise dadurch ausführen, daß der in dem weiteren Speicher abgespeicherte Signalverlauf jedesmal durch den zwischengespeicherten zeitlichen Verlauf des elektrischen Gewebepotentials ersetzt wird.

Alternativ hierzu ist entsprechend einer vorteilhaften Weiterbildung der erfindungsgemäßen Anordnung dem ersten Speicher und dem weiteren Speicher ein Mittelwertbildner zugeordnet, in dem zur Aktualisierung des in dem weiteren Speicher abgespeicherten Signalverlaufs dieser und der in dem ersten Speicher zwischengespeicherte zeitliche Verlauf des Gewebepotentials zu einem Mittelwertsignal verknüpft werden, das in dem weiteren Speicher abgespeichert wird.

Die Auswertung des Differenzsignals läßt sich auf technisch einfachste Weise dadurch ausführen, daß die Auswerteeinrichtung einen Schwellenwertdetektor zur Überwachung des Differenzsignals auf Überschreiten eines vorgegebenen Schwellenwertes aufweist.

Um bei der Auswertung des Differenzsignals außer seiner Amplitude auch seine Signalbreite berücksichtigen zu können weist entsprechend einer vorteilhaften Weiterbildung der erfindungsgemäßen Anordnung die Auswerteeinrichtung einen Integrator zur Integration des Differenzsignals und einen nachgeordneten Schwellenwertdetektor zur Überwachung des integrierten Differenzsignals auf Überschreiten eines vorgegebenen Schwellenwertes auf.

Eine von dem jeweiligen Bezugspotential (Nullinie) des Differenzsignals unabhängige Auswertung wird in vorteilhafter Weise dadurch ermöglicht, daß dem Schwellenwertdetektor ein Spitzenwertspeicher zur Erfassung des Spitze-Spitze-Wertes des Differenzsignals bzw. des integrierten Differenzsignals vorgeordnet ist.

Obwohl bei der erfindungsgemäßen Anordnung der zur Kompensation der Polarisationskomponente in dem erfaßten elektrischen Potentialverlauf herangezogene abgespeicherte Signalverlauf ständig aktualisiert wird, ist er jedoch mit der Polarisationskomponente in dem erfaßten elektrischen Potentialverlauf nicht identisch. Da die Polarisationserscheinungen eine vielfach höhere Amplitude als die zu detektierende potentielle Stimulationsantwort aufweisen, können daher bereits geringe prozentuale Unterschiede zwischen der Polarisationskomponente des aktuell erfaßten Potentialverlauf und dem abgespeicherten Signalverlauf die Auswertung des Differenzsignals erschweren. Entsprechend einer vorteilhaften Weiterbildung der erfindungsgemäßen Anordnung werden daher die Polarisationserscheinungen von vorneherein dadurch reduziert, daß der Stimulationsimpulsgenerator biphasische Stimulationsimpulse erzeugt.

Zur Erläuterung der Erfindung wird im folgenden auf die Figuren der Zeichnung Bezug genommen; im einzelnen zeigen

FIG 1 ein Blockschaltbild eines Ausführungsbeispiels der erfindungsgemäßen Anordnung,

FIG 2 in einem Zeitdiagramm Beispiele für den Verlauf des elektrischen Gewebepotentials bei einem erfolglosen und einem erfolgreichen Stimulationsversuch und ein daraus gebildetes Differenzsignal, die

FIG 3 und 4 jeweils ein Ausführungsbeispiel für die Detektoreinrichtung der in FIG 1 gezeigten Anordnung und die

FIG 5 bis 8 jeweils ein Ausführungsbeispiel für die in den FIG 3 und 4 gezeigte Auswerteeinrichtung.

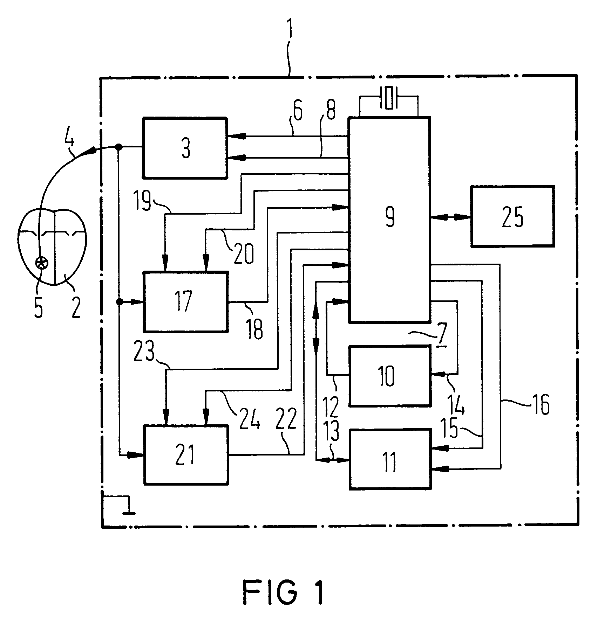

FIG 1 zeigt als Ausführungsbeispiel für die erfindungsgemäße Anordnung das Blockschaltbild eines Herzschrittmachers (1) zur Gewebestimulation, in diesem Fall der Stimulation eines Herzens (2). Der Herzschrittmacher (1) enthält einen Stimulationsimpulsgenerator (3), der zur Beaufschlagung des Herzens (2) mit Stimulationsimpulsen ausgangsseitig über eine Elektrodenleitung (4) mit einer im Herzen (2) applizierten Elektrode (5) verbunden ist. Der Stimulationsimpulsgenerator (3) ist über eine Steuerleitung (6), die mit einem entsprechenden Ausgang einer Steuereinrichtung (7) verbunden ist, zur Abgabe eines Stimulationsimpulses aktivierbar, wobei über dieselbe Leitung (6) auch die Dauer des jeweiligen Stimulationsimpulses einstellbar ist. Zur Einstellung der Amplitude der Stimulationsimpulse ist der Stimulationsimpulsgenerator (3) über eine weitere Steuerleitung (8) mit der Steuereinrichtung (7) verbunden. Die Steuereinrichtung (7) besteht bei dem dargestellten Ausführungsbeispiel aus einem Mikroprozessor (9), dem ein Nur-Lese-Speicher (ROM) (10) und ein Schreib-Lese-Speicher (RAM) (11) zugeordnet ist, die über Datenleitungen (12,13) und Adressenleitungen (14,15) sowie im Falle des Schreib-Lese-Speichers (11) zusätzlich über eine Schreib-Lese-Umschaltleitung (16) mit dem Mikroprozessor (9) verbunden sind. In dem Nur-Lese-Speicher (10) ist ein Programm gespeichert, das sämtliche Funktionen des Herzschrittmachers (1) über den Mikroprozessor (9) ausführt.

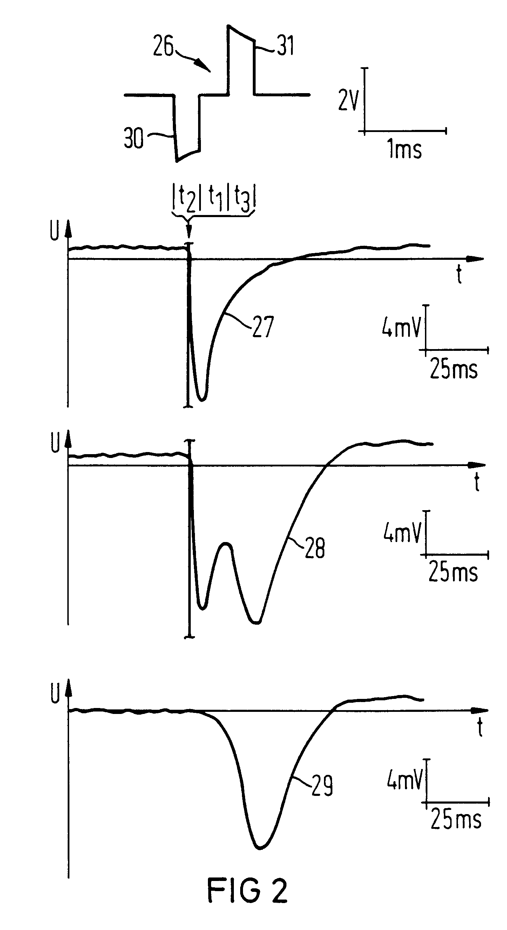

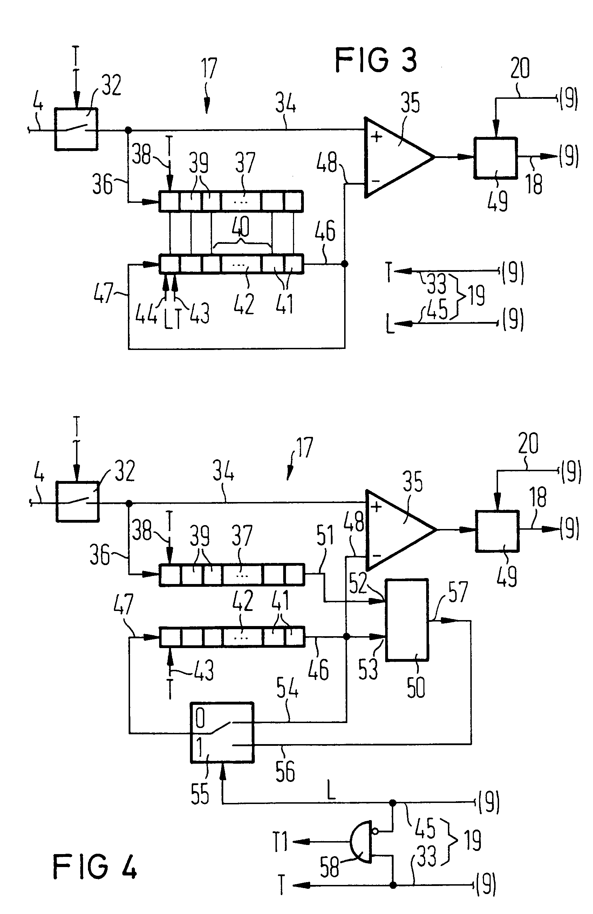

Um bei einer Stimulation die Reaktion des Herzens (2) erfassen zu können, enthält der Herzschrittmacher (1) eine Detektoreinrichtung (17), die eingangsseitig zur Erfassung des elektrischen Potentials im Herzgewebe über die Elektrodenleitung (4) mit der Elektrode (5) verbunden ist. Diese Anordnung ist besonders einfach, weil sowohl zur Stimulation des Herzens (2) als auch zur Erfassung seiner Reaktion (Stimulationsantwort) nur eine einzige Elektrode (5) benötigt wird. Dabei entsteht jedoch das Problem, daß bei jeder Stimulation das Herzgewebe im unmittelbaren Bereich der Elektrode (5) dermaßen stark polarisiert wird, daß die Polarisationseffekte die Stimulationsantwort des Herzens (2) bis zur Unkenntlichkeit überlagern. Dieses Problem und seine erfindungsgemäße Lösung wird untenstehend anhand von FIG 2 näher erläutert. Das Ausführungsbeispiel der erfindungsgemäßen Anordnung nach FIG 1 läßt natürlich auch die Verwendung einer getrennten Stimulationselektrode und Meßelektrode zur Stimulation bzw. zum Erfassen der Stimulationsantwort zu. Der von der Detektoreinrichtung (17) erfaßte und auf das Vorhandensein oder Fehlen einer Stimulationsantwort ausgewertete elektrische Potentialverlauf im Herzgewebe wird über eine Ausgangsleitung (18) der Detektoreinrichtung (17) einem entsprechenden Eingang des Mikroprozessors (9) zugeführt, der bei fehlender Stimulationsantwort eine Erhöhung, anderenfalls eine Verringerung der Stimulationsenergie veranlaßt. Die Detektoreinrichtung (17) wird von dem Mikroprozessor (9) über eine ggf. aus mehreren Einzelleitungen bestehende Steuerleitung (19) zur Erfassung des Potentialverlaufs im Herzen (2) aktiviert bzw. gesperrt und über eine weitere Steuerleitung (20) bezüglich ihrer Empfindlichkeit eingestellt. Der Aufbau der Detektoreinrichtung (17) wird untenstehend anhand der FIG 3 bis 8 näher erläutert.

Zur Detektion natürlicher Herzaktivitäten enthält der Herzschrittmacher (1) eine weitere Detektoreinrichtung (21), die ebenfalls eingangsseitig über die Elektrodenleitung (4) mit der Elektrode (5) verbunden ist und über eine Ausgangsleitung (22) an dem Mikroprozessor (9) angeschlossen ist. Die weitere Detektoreinrichtung (21) ist über Steuerleitungen (23,24) durch den Mikroprozessor (9) aktivierbar oder abschaltbar und bezüglich ihrer Empfindlichkeit einstellbar. Die weitere Detektoreinrichtung (21) dient dazu, natürliche Herzschläge zu erfassen und über den Mikroprozessor (9) eine Stimulation des Herzens (2) solange zu unterbinden, wie eine natürlich Herzaktivität vorliegt; diese Funktion ist an sich bekannt und wird daher im folgenden nicht weiter erläutert.

Schließlich enthält der Herzschrittmacher (1) eine mit dem Mikroprozessor (9) verbundene Telemetrieeinrichtung (25) zum Programmieren und Überwachen von Funktionen des Herzschrittmachers (1) und von diesem erfaßten Herzparametern durch Datenaustausch mit einem äußeren, hier nicht gezeigten Programmier- und Überwachungsgerät.

FIG 2 zeigt in einem Zeitdiagramm von oben nach unten einen von dem Stimulationsimpulsgenerator (3) erzeugten und an das Herz (2) abgegebenen Stimulationsimpuls (26), einen von dem Stimulationsimpuls (26) hervorgerufenen Verlauf des elektrischen Potentials (27) im Herzgewebe bei einer unterhalb der Reizschwelle des zu stimulierenden Herzgewebes liegenden Stimulationsenergie, einen Verlauf des elektrischen Gewebepotentials (28) bei einer Stimulationsenergie oberhalb der Reizschwelle und ein aus den beiden Potentialverläufen (27,28) gebildetes Differenzsignal (29). Aus Gründen der besseren Darstellbarkeit des Stimulationsimpulses (26) ist dessen Amplitude im Vergleich zu den Kurvenverläufen (27,28,29) gestaucht und dessen Zeitdauer gedehnt dargestellt; auf Grund des anderen Darstellungsmaßstabes erscheint der Stimulationsimpuls (26) in den Verläufen des Gewebepotentials (27,28) jeweils als senkrechter Strich.

Bei dem dargestellten Stimulationsimpuls (26) handelt es sich um einen biphasischen Impuls, der aus einem negativen Teilimpuls (30) und einem auf diesen nach einer Impulspause $t_1$ folgenden positiven Teilimpuls (31) besteht. Die Impulsamplituden und Impulsdauern $t_2$ und $t_3$ der Teilimpulse (30,31) sind jeweils vorzugsweise gleich, wobei durch Änderung der Impulsamplituden und/oder der Impulsdauern $t_2$ und $t_3$ eine Variation der Stimulationsenergie möglich ist. Der Vorteil des biphasischen Stimulationsimpulses (26) gegenüber einem im Rahmen der Erfindung ebenfalls anwendbaren monophasischen Stimulationsimpuls besteht in der relativ schnellen Reduzierung der durch die Stimulation im Gewebe hervorgerufenen Polarisationserscheinungen durch den auf den stimulationsaulösenden negativen Teilimpuls (30) folgenden positiven Teilimpuls (31), so daß im Falle einer erfolgreichen Stimulation die Erfassung der Reaktion des Gewebes auf die Stimulation (Stimulationsantwort) erleichtert wird.

Der mit (27) bezeichnete Verlauf des elektrischen Potentials im Gewebe ergibt sich bei einem Stimulationsversuch mit dem Stimulationsimpuls (26) und einer unterhalb der Reizschwelle liegenden Stimulationsenergie, wobei der exponentiell abklingende Kurvenabschnitt den durch den Stimulationsimpuls (26) im Gewebe hervorgerufenen Polarisationserscheinungen entspricht. Auf Grund der Bipolarität des Stimulationsimpulses (26) sind die Polarisationserscheinungen im Vergleich mit einem unipolaren Stimulationsimpuls erheblich reduziert.

Der mit (28) bezeichnete Verlauf des elektrischen Gewebepotentials ergibt sich bei Stimulation mit einer über der Reizschwelle des Herzgewebes liegenden Stimulationsenergie und zeigt auch hier die exponentiell abklingenden Polarisationserscheinungen auf, die dieses Mal von der Stimulationsantwort des Herzens (2) überlagert sind. Die Stimulationsantwort ist hier lediglich auf Grund der polarisationsreduzierenden Wirkung des biphasischen Stimulationsimpulses (26) sichtbar und wäre im Falle einer monophasischen Stimulation bis zur Unkenntlichkeit von den Polarisationserscheinungen überlagert. Durch die Bildung des Differenzsignales (29) aus den Kurvenverläufen (27,28) wird jedoch die Stimulationsantwort aus dem Verlauf des Gewebepotentials (28) herausgefiltert und zwar im Prinzip auch dann, wenn die Polarisationserscheinungen nicht durch bestimmte Maßnahmen, wie z.B. die Verwendung biphasischer Stimulationsimpulse (26) reduziert worden sind. Da jedoch die Polarisationserscheinungen eine im Vergleich zur Stimulationsantwort große Signalkomponente bilden, können auch relativ kleine prozentuale Abweichungen in den Kurvenverläufen (27,28) zu einer in dem Differenzsignal (29) verbleibenden Restkomponente führen, durch die die Detektion der Stimulationsantwort in dem Differenzsignal (29) erschwert wird. Daher ist eine Reduzierung der Polarisationserscheinungen von vornherein auch im Rahmen der Erfindung vorteilhaft.

FIG 3 zeigt ein erstes Ausführungsbeispiel für die in FIG 1 gezeigte Detektoreinrichtung (17) zur Bildung und Auswertung des in FIG 2 gezeigten Differenzsignales (29). Die Detektoreinrichtung (17) enthält eingangsseitig eine über die Elektrodenleitung (4) mit der Elektrode (5) verbundene Abtasteinrichtung (32), die von dem Mikroprozessor (9) über eine Taktsteuerleitung (33) mit einem Abtasttakt T steuerbar ist. Die Abtasteinrichtung (32) ist ausgangsseitig sowohl mit einem ersten Eingang (34) einer Subtrahiereinrichtung (35) als auch mit einem seriellen Dateneingang (36) eines ersten Speichers (37) verbunden. Der erste Speicher (37) ist an einem Taktsteuereingang (38) mit der Taktsteuerleitung (33) verbunden und weist eine vorgegebene Anzahl nebeneinanderliegender Speicherplätze (39) auf, so daß ein dem ersten Speicher (37) über den Dateneingang (36) zugeführter Abtastwert bei jedem Takt T von einem Speicherplatz (39) zu dem nächsten Speicherplatz (39) verschoben wird. Die Speicherplätze (39) sind über parallele Datenausgangsleitungen (40) mit entsprechenden Speicherplätzen (41) eines weiteren Speichers (42) verbunden, der ebenfalls an einem Taktsteuereingang (43) mit der Taktsteuerleitung (33) verbunden ist. Ein weiterer Steuereingang (44) des weiteren Speichers (42) ist über eine Datenübernahmesignalleitung (45) von dem Mikroprozessor (9) mit einem Datenübernahmesignal L beaufschlagbar, um die Abtastwerte aus dem ersten Speicher (37) über die parallelen Datenleitungen (40) in den weiteren Speicher (42) zu kopieren. Die beiden mit (33) und (45) bezeichneten Leitungen bilden zusammen die in FIG 1 mit (19) bezeichnete Steuerleitung. Der weitere Speicher (42) ist als Umlaufspeicher ausgebildet, indem von den nebeneinanderliegenden Speicherplätzen (41) der letzte Speicherplatz (41) über einen seriellen Datenausgang (46) mit einem seriellen Dateneingang (47) des ersten Speicherplatzes (41) verbunden ist. Der serielle Datenausgang (46) ist außerdem mit einem zweiten Eingang (48) der Subtrahiereinrichtung (35) verbunden. Die Subtrahiereinrichtung (35) ist ausgangsseitig an einer Auswerteeinrichtung (49) angeschlossen, die an ihrem Ausgang über die Ausgangsleitung (18) mit dem Mikroprozessor (9) verbunden ist. Die Empfindlichkeit der Auswerteeinrichtung (49) läßt sich über die weitere Steuerleitung (20) durch den Mikroprozessor (9) einstellen.

Zur Erläuterung der Funktionsweise der in FIG 3 gezeigten Detektoreinrichtung (17) sei angenommen, daß in dem weiteren Speicher (42) die Abtastwerte eines Signalverlaufs abgespeichert sind, der die durch die Stimulation im Herzgewebe hervorgerufenen Polarisationserscheinungen repräsentiert. Dies kann beispielsweise dadurch geschehen, daß das Herz (2) durch den Stimulationsimpulsgenerator (3) mit einem Stimulationsimpuls (26) beaufschlagt wird, dessen Stimulationsenergie unterhalb der Reizschwelle des Herzgewebes liegt; unmittelbar nach der Stimulation erzeugt der Mikroprozessor (9) für eine vorgegebene Zeitdauer den Takt T, mit dem das elektrische Potential im Herzgewebe in einem auf die Stimulation folgenden Zeitfenster von 40-200 ms durch die Abtasteinrichtung (32) abgetastet wird. Die Abtastwerte werden der Reihe nach in die Speicherplätze (39) des ersten Speichers (37) eingelesen und dort zwischengespeichert. Anschließend erzeugt der Mikroprozessor (9) das Datenübernahmesignal L, woraufhin die Abtastwerte aus den Speicherplätzen (39) des ersten Speichers (37) in die zugeordneten Speicherplätze (41) des weiteren Speichers (42) kopiert werden. In dem weiteren Speicher (42) liegen nun Abtastwerte eines Signalverlaufs vor, der dem in FIG 2 mit (27) bezeichneten Kurvenverlauf entspricht und die durch die Stimulation im Gewebe hervorgerufenen Polarisationserscheinungen repräsentiert. Es besteht aber auch die Möglichkeit, die Speicherplätze (41) des weiteren Speichers (42) mit Startwerten zu laden, die in dem Mikroprozessor (9) nach einer den Verlauf der Polarisationserscheinungen annähernd wiedergebenden Formel berechnet werden oder aus dem Nur-Lese-Speicher (10) stammen.

Zur Überwachung der Stimulation des Herzens (2) wird nach der Abgabe eines Stimulationsimpulses (26) das elektrische Potential im Herzgewebe in dem oben genannten Zeitfenster mit Hilfe der Abtasteinrichtung (32) abgetastet. Die so gewonnenen Abtastwerte werden nacheinander dem ersten Eingang (34) der Subtrahiereinrichtung (35) zugeführt und gleichzeitig in dem ersten Speicher (37) zwischengespeichert. Dazu synchron werden die in dem weiteren Speicher (42) gespeicherten Abtastwerte der Polarisationserscheinungen dem zweiten Eingang (48) der Subtahiereinrichtung (35) zugeführt, wobei dieselben Abtastwerte wieder vorne in den zweiten Speicher (42) eingelesen werden, so daß sie nicht verloren gehen. Für den Fall, daß die Stimulationsenergie über der Reizschwelle des Herzgewebes lag, liegen an dem ersten Eingang (34) der Subtrahiereinrichtung (35) die Abtastwerte des in FIG 2 mit (28) bezeichneten Kurvenverlaufes an, während an dem zweiten Eingang die Abtastwerte des Kurvenverlaufes (27) anliegen. In der Subtrahiereinrichtung (35) werden diese beiden Kurvenverläufe (27,28) voneinander subtrahiert, so daß der Auswerteeinrichtung (49) der in FIG 2 mit (29) bezeichnete und die Stimulationsantwort wiedergebende Signalverlauf zugeführt wird. Für den Fall, daß die Stimulationsenergie unter der Reizschwelle des Herzgewebes lag, werden dem ersten Eingang (34) der Subtrahiereinrichtung (35) die Abtastwerte eines weitgehend der Kurve (27) entsprechenden Potentialverlaufs zugeführt. Da sich diese Abtastwerte von denen in dem weiteren Speicher (42) gespeicherten Abtastwerten nur unwesentlich unterscheiden, entsteht an dem Ausgang der Subtrahiereinrichtung (35) ein Differenzsignal mit allenfalls nur geringer Amplitude, was in der Auswerteeinrichtung (49) als Fehlen einer Stimulationsantwort gewertet wird und dem Mikroprozessor über die Ausgangsleitung (18) mitgeteilt wird. Der Mikroprozessor (9) veranlaßt daraufhin durch Erzeugung des Datenübernahmesignals L eine Übernahme der in dem ersten Speicher (37) zwischengespeicherten Abtastwerte in den weiteren Speicher (42). Auf diese Weise wird bei jedem von der Auswerteeinrichtung (49) detektierten Fehlen einer Reaktion des Herzgewebes auf die Stimulation der in dem weiteren Speicher (42) abgespeicherte Verlauf der Polarisationserscheinungen aktualisiert.

Das in FIG 4 gezeigte Ausführungsbeispiel für die Detektoreinrichtung (17) unterscheidet sich von dem Ausführungsbeispiel nach FIG 3 dadurch, daß zur Aktualisierung des in dem weiteren Speicher (42) abgespeicherten Signalverlaufs dieser nicht einfach durch den in dem ersten Speicher (37) zwischengespeicherten Verlauf des elektrischen Gewebepotentials ersetzt wird, sondern daß beide in den Speichern (37,42) abgespeicherten Signalverläufe einem Mittelwertbildner (50) zugeführt werden, in dem die beiden Signalverläufe zu einem Mittelwertsignal verknüpft werden, das danach in dem weiteren Speicher (42) abgespeichert wird. Die in FIG 4 gezeigte Detektoreinrichtung (17) weist also eine mit einem Abtasttakt T1 steuerbare Abtasteinrichtung (32) auf, die eingangsseitig über die Elektrodenleitung (4) mit der Elektrode (5) im Herzen verbunden ist und ausgangsseitig an dem ersten Eingang (34) einer Subtrahiereinrichtung (35) angeschlossen ist. Der Subtrahiereinrichtung (35) ist wiederum eine Auswerteeinrichtung (49) nachgeordnet, die über die Ausgangsleitung (18) und die weitere Steuerleitung (20) mit dem Mikroprozessor (9) verbunden ist. Die Abtasteinrichtung (32) ist ausgangsseitig weiterhin mit einem seriellen Dateneingang (36) des ersten Speichers (37) verbunden, der im Unterschied zu dem Ausführungsbeispiel nach FIG 3 anstelle von parallelen Datenausgangsleitungen einen seriellen Datenausgang (51) an dem letzten der Speicherplätze (39) aufweist. Der serielle Datenausgang (51) ist mit einem ersten Eingang (52) des Mittelwertbildners (50) verbunden. Der weitere Speicher (42), der eine gleiche Anzahl von Speicherplätzen (41) wie der erste Speicher (37) aufweist, ist an seinem seriellen Datenausgang (46) sowohl an dem zweiten Eingang (48) der Subtrahiereinrichtung (35) als auch an einem zweiten Eingang (53) des Mittelwertbildners (50) angeschlossen und zusätzlich mit einem ersten Umschaltkontakt (54) eines steuerbaren Umschalters (55) verbunden, dessen zweiter Umschaltkontakt (56) mit einem Ausgang (57) des Mittelwertbildners (50) verbunden ist. Der Umschalter (55) ist von dem Mikroprozessor (9) über die Einzelleitung (45) mit dem Signal L derart steuerbar, daß bei L = 0 der erste Umschaltkontakt (54) mit einem seriellen Dateneingang (47) des weiteren Speichers (42) verbunden wird und daß bei L = 1 der zweite Umschaltkontakt (56) mit dem seriellen Dateneingang (47) verbunden wird. Im Unterschied zu dem Takt T für die beiden Speicher (37,42) wird der Abtasttakt T1 für die Abtasteinrichtung (32) in einem Verknüpfungsglied (58) nach der logischen Verknüpfung $T1 = T \cdot \overline{L}$ erzeugt.

Bei der Überwachung der Stimulation des Herzens (2) befindet sich der Umschalter (55) in der mit "0" bezeichneten Umschaltstellung, so daß gleichzeitig mit den von der Abtasteinrichtung (32) kommenden und dem ersten Eingang (34) der Vergleichseinrichtung (35) zugeführten Abtastwerten, die in dem weiteren Speicher (42) abgespeicherten Werte sowohl dem zweiten Eingang (48) der Vergleichseinrichtung (35) zugeführt werden als auch über den Umschalter (55) wieder vorne in den weiteren Speicher (42) eingelesen werden. Die der Vergleichseinrichtung (35) über ihre Eingänge (34,48) zugeführten Signalverläufe werden in derselben Weise wie anhand von FIG 3 bereits beschrieben voneinander subtrahiert, wobei das so erhaltene Differenzsignal in der Auswerteeinrichtung (49) auf das Vorhandensein oder Fehlen einer Stimulationsantwort überprüft wird. Beim Fehlen einer Stimulationsantwort wird der Umschalter (55) in die mit "1" bezeichnete Umschaltstellung gesteuert und die in dem ersten Speicher (37) und in dem weiteren Speicher (42) abgespeicherten Abtastwerte paarweise dem Mittelwertbildner (50) zugeführt, wobei die von dem Mittelwertbildner (50) erzeugten Mittelwerte über den Umschalter (55) dem weiteren Speicher (52) wieder zugeführt werden. Dabei werden die beiden Speicher (37,41) durch den Takt T gesteuert, während der Abtasttakt T1 für die Abtasteinrichtung (32) auf Grund von L = 1 gesperrt ist.

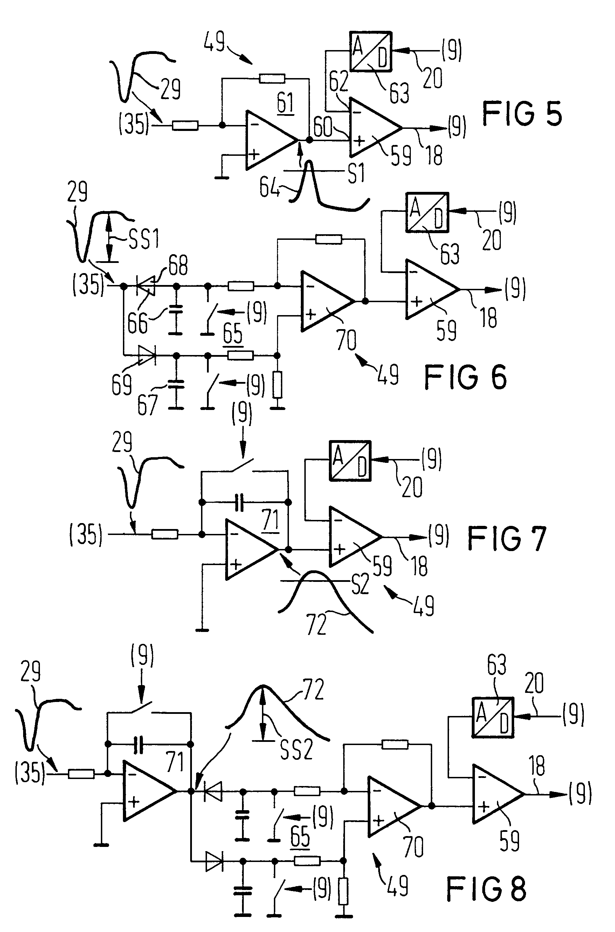

Im folgenden wird die Auswertung des von der Subtrahiereinrichtung (35) erzeugten Differenzsignales (29) in der Auswerteeinrichtung (49) anhand von mehreren Ausführungsbeispielen für diese Auswerteeinrichtung (49) erläutert. Wie obenstehend bereits erläutert, unterscheidet sich das Differenzsignal am Ausgang der Subtrahiereinrichtung (35) beim Vorliegen einer Stimulationsantwort von dem Differenzsignal beim Fehlen einer Stimulationsantwort durch die wesentlich höhere Amplitude. Im einfachsten Fall besteht daher die Auswerteeinrichtung (49) entsprechend dem Ausführungsbeispiel nach FIG 5 aus einem Schwellenwertkomparator (59), dessen nichtinvertierender Eingang (60) direkt oder wie hier gezeigt über einen invertierenden Verstärker (61) mit dem Ausgang der Subtrahiereinrichtung (35) verbunden ist und dessen invertierenden Eingang (62) von dem Mikroprozessor (9) über einen Digital-/Analog-Umsetzer (63) ein Schwellenwert S1 zugeführt wird. Übersteigt das invertierte Differenzsignal (64) den Schwellenwert S1, so erzeugt der Schwellenwertkomparator (59) ein Ausgangssignal, das dem Mikroprozessor (9) die Detektion einer Stimulationsantwort anzeigt.

Das in FIG 6 gezeigte Ausführungsbeispiel der Auswerteeinrichtung (49) unterscheidet sich von dem Ausführungsbeispiel nach FIG 5 dadurch, daß der invertierende Verstärker (61) durch einen Spitzenwertspeicher (65) ersetzt ist, der den Spitze-Spitze-Wert SS1 zwischen dem Minimum und Maximum des Differenzsignals (29) in dem verwendeten Abtast-Zeitfenster erfaßt. Der Spitzenwertspeicher (65) besteht aus zwei Kondensatoren (66,67), die über unterschiedlich gepolte Dioden (68,69) mit positiven bzw. negativen Scheitelwert des dem Differenzsignals (29) aufgeladen werden; die beiden Scheitelwerte werden in einem Addierer (70) zu dem Spitze-Spitze-Wert SS1 aufaddiert, der in dem nachgeordneten Schwellenwertkomparator (59) auf das Überschreiten eines Schwellenwertes überwacht wird. Die Auswertung des Differenzsignals (29) ist hierbei unabhängig von dessen jeweiligen Bezugspotential (Nulllinie). Nach jeder Auswertung werden die in den Kondensatoren (66,67) gespeicherten Scheitelwerte durch Kurzschließen der Kondensatoren (66,67) gelöscht.

Bei dem Ausführungsbeispiel nach FIG 7 ist der Schwellenwertkomparator (59) eingangsseitig über einen invertierenden Integrator (71) mit dem Ausgang der Subtrahiereinrichtung (35) verbunden. Durch die Integration des Differenzsignals (29) und Überwachung des Integrationssignals (72) auf Überschreiten einer Schwelle S2 wird bei der Auswertung des Differenzsignals (29) nicht nur seine Amplitude sondern auch seine Breite berücksichtigt. Nach jeder Auswertung wird der Integrator (71) auf einen Anfangswert zurückgesetzt.

Bei dem in FIG 8 gezeigten Ausführungsbeispiel für die Auswerteeinrichtung (49) sind der Integrator (71), der Spitzenwertspeicher (65) und der Schwellenwertkomparator (59) in Reihe an den Ausgang der Subtrahiereinrichtung (35) angeschlossen. Bei der Auswertung des Differenzsignals (29) wird daher sowohl dessen Amplitude als auch dessen Breite erfaßt, wobei das jeweilige Bezugspotential (Nullinie) des Differenzsignals (29) keinen Einfluß auf die Auswertung hat.

Die Genauigkeit der Überwachung der Stimulation läßt sich noch weiter erhöhen, indem von dem im Herzgewebe erfaßten elektrischen Potential nur diejenigen Signalanteile zur Auswertung in der Detektoreinrichtung (17) gelangen, die hinsichtlich ihrer Frequenz und Steilheit für stimulierte Gewebereaktionen typisch sind. Aus diesem Grund wird der von der Detektoreinrichtung (17) erfaßte Potentialverlauf - wegen des Abtasttheorems nach Shannon vorzugsweise vor der Abtasteinrichtung (32) - durch ein Bandpaßfilter frequenzbegrenzt. Da eine derartige Frequenzbegrenzung ebenso wie eine Meßverstärkung bei der Erfassung physiologischer Signale allgemein üblich sind, wurde auf eine entsprechende Darstellung in den Figuren verzichtet.

Sämtliche in Verbindung mit den Figuren gezeigten und erläuterten Möglichkeiten der Signalauswertung lassen sich im Rahmen der Erfindung auch durch Programmablauf in dem Mikroprozessor (9) ausführen, indem der im Herzgewebe erfaßte Potentialverlauf entweder unmittelbar nach seiner Erfassung oder nach einer Teilauswertung in Digitalwerte umgesetzt dem Mikroprozessor zugeführt wird.

## Patentansprüche

1. Anordnung zur Gewebestimulation bei einem Lebewesen mit einem Stimulationsimpulsgenerator (3) zur Erzeugung von Stimulationsimpulsen (26), mit einer an dem Stimulationsimpulsgenerator (3) angeschlossenen Elektrodenanordnung (4,5) zur Abgabe der Stimulationsimpulse (26) an das Gewebe, mit einer ein elektrisches Potential (27,28) im Gewebe erfassenden Detektoreinrichtung (17), die eine Subtrahiereinrichtung (35) zur Erzeugung eines Differenzsignales (29) aus dem erfaßten elektrischen Potential (28) und einem durch die Stimulation im Gewebe hervorgerufene Polarisationserscheinungen repräsentierenden Signalverlauf (27) enthält und eine der Subtrahiereinrichtung (35) nachgeordnete Auswerteeinrichtung (49) zur Auswertung des Differenzsignales (29) auf das Vorhandensein oder Fehlen einer Reaktion des Gewebes als Folge der Stimulation aufweist, und mit einer Steuereinrichtung (7) zur Steuerung des Stimulationsimpulsgenerators (3) in Abhängigkeit von dem Ergebnis der Auswertung des Differenzsignales (29), **dadurch gekennzeichnet,** daß die Anordnung einen ersten Speicher (37) zur Zwischenspeicherung des nach einer Stimulation von der Detektoreinrichtung (17) erfaßten zeitlichen Verlaufs des elektrischen Gewebepotentials (28) aufweist, daß der die Polarisationserscheinungen repräsentierende Signalverlauf (27) in einem weiteren Speicher (42) abgespeichert ist und daß bei einem von der Auswerteeinrichtung (49) detektierten Fehlen einer Reaktion des Gewebes auf die Stimulation der zwischengespeicherte zeitliche Verlauf des elektrischen Gewebepotentials zur Aktualisierung des Signalverlaufs (27) in dem weiteren Speicher (42) herangezogen wird.

2. Anordnung nach Anspruch 1, **dadurch gekennzeichnet,** daß zur Aktualisierung des in dem weiteren Speicher (42) abgespeicherten Signalverlaufs (27) dieser durch den zwischengespeicherten zeitlichen Verlauf des elektrischen Gewebepotentials ersetzt wird.

3. Anordnung nach Anspruch 1, **dadurch gekennzeichnet,** daß dem ersten Speicher (37) und dem weiteren Speicher (42) ein Mittelwertbildner (50) zugeordnet ist, in dem zur Aktualisierung des in dem weiteren Speicher (42) abgespeicherten Signalverlaufs dieser und der in dem ersten Speicher (37) zwischengespeicherte zeitliche Verlauf des Gewebepotentials zu einem Mittelwertsignal verknüpft werden, das in dem weiteren Speicher (42) abgespeichert wird.

4. Anordnung nach einem der vorangehenden Ansprüche, **dadurch gekennzeichnet,** daß die Auswerteeinrichtung einen Schwellenwertdetektor (59) zur Überwachung des Differenzsignals (29) auf Überschreiten eines vorgegebenen Schwellenwertes S1 aufweist.

5. Anordnung nach einem der Ansprüche 1 bis 3, **dadurch gekennzeichnet,** daß die Auswerteeinrichtung (49) einen Integrator (71) zur Integration des Differenzsignals (29) und einen nachgeordneten Schwellenwertdetektor (59) zur Überwachung des integrierten Differenzsignals (72) auf Überschreiten eines vorgegebenen Schwellenwertes S2 aufweist.

6. Anordnung nach Anspruch 4 oder 5, **dadurch gekennzeichnet,** daß dem Schwellenwertdetektor (59) ein Spitzenwertspeicher (65) zur Erfassung des Spitze-Spitze-Wertes SS1 bzw. SS2 des Differenzsignals (29) bzw. des integrierten Differenzsignals (72) vorgeordnet ist.

7. Anordnung nach einem der vorangehenden Ansprüche, **dadurch gekennzeichnet,** daß der Stimulationsimpulsgenerator (3) biphasische Stimulationsimpulse (26) erzeugt.

## Claims

1. Arrangement for tissue stimulation in a living organism, having a stimulation pulse generator (3) for generating stimulation pulses (26), having an electrode arrangement (4, 5), connected to the stimulation pulse generator 93), for emitting stimulation pulses (26) to the tissue, having a detector device (17), which picks up an electrical potential (27, 28) in the tissue and which exhibits a subtractor device (35) for generating a difference signal (29) from the picked-up electrical potential (28) and a signal progression (27) representing polarization phenomena caused by the stimulation in the tissue and an evaluating device (49), connected downstream of the subtractor device (35), for evaluating the difference signal (29) for the presence or absence of a reaction of the tissue as a consequence of the stimulation, and having a control device (7) for controlling the stimulation pulse generator (3) as a function of the result of the evaluation of the difference signal (29), characterized in that the arrangement exhibits a first memory (37) for the intermediate storage of the temporal progression of the electrical tissue potential (28) picked up by the detector device (17) after a stimulation, in that the signal progression (27) representing the polarization phenomena is stored in a further memory (42), and in that in the event of the evaluating device (49) detecting the absence of a reaction of the tissue to the stimulation, the temporal progression, placed in intermediate storage, of the electrical tissue potential is utilized to update the signal progression (27) in the further memory (42).

2. Arrangement according to Claim 1, characterized in that in order to update the signal progression (27) stored in the further memory (42), said signal progression is replaced by the temporal progression, placed in intermediate storage, of the electrical tissue potential.

3. Arrangement according to Claim 1, characterized in that with the first memory (37) and the further memory (42) there is associated a mean-value former (50), in which, in order to update the signal progression stored in the further memory (42), said signal progression and the temporal progression, placed in intermediate storage in the first memory (37), of the tissue potential are linked to form a mean-value signal, which is stored in the further memory (42).

4. Arrangement according to one of the preceding claims, characterized in that the evaluating device exhibits a threshold-value detector (59) to monitor the difference signal (29) for the exceeding of a predetermined threshold value S1.

5. Arrangement according to one of Claims 1 to 3, characterized in that the evaluating device (49) exhibits an integrator (71) for integrating the difference signal (29) and a downstream threshold-value detector (59) for monitoring the integrated difference signal (72) for the exceeding of a predetermined threshold value S2.

6. Arrangement according to Claim 4 or 5, characterized in that a peak value memory (65) to pick up the peak-peak value SS1 or SS2 respectively of the difference signal (29) or respectively of the integrated difference signal (72) is disposed upstream of the threshold-value detector (59).

7. Arrangement according to one of the preceding claims, characterized in that the stimulation pulse generator (3) generates bi-phase stimulation pulses (26).

## Revendications

1. Dispositif de stimulation de tissus chez un être vivant, comportant un générateur d'impulsions de stimulation (3) servant à produire des impulsions de stimulation (26), un dispositif à électrodes (4,5), qui est raccordé au générateur d'impulsions de stimulation (3) et qui sert à envoyer les impulsions de stimulation (26) au tissu, un dispositif détecteur (17), qui détecte un potentiel électrique (27,28) dans le tissu et qui comporte un dispositif soustracteur (35) servant à produire un signal de différence (22) à partir du potentiel électrique détecté (28) et d'une variation de signal (27), qui représente des phénomènes de polarisation produits par la stimulation dans le tissu, et un dispositif d'exploitation (49), qui est disposé en aval du dispositif soustracteur (35) et qui sert à exploiter le signal de différence (29) pour déterminer la présence ou l'absence d'une réaction du tissu à la suite de la stimulation, et un dispositif de commande (7) servant à commander le générateur d'impulsions de stimulation (3) en fonction du résultat de l'exploitation du signal de différence (29), caractérisé par le fait que le dispositif comporte une première mémoire (37) pour la mémorisation temporaire de la variation dans le temps, détectée par le dispositif détecteur (17) après une stimulation, du potentiel électrique (28) du tissu, que la variation du signal (27), qui représente les phénomènes de polarisation, est mémorisée dans une autre mémoire (42) et que, dans le cas de l'absence, détectée par le dispositif d'exploitation (49), d'une réaction du tissu à la stimulation, la variation dans le temps, mémorisée temporairement, du potentiel électrique du tissu est utilisée pour l'actualisation de la variation du signal (27) dans l'autre mémoire (42).

2. Dispositif suivant la revendication 1, caractérisé par le fait que pour l'actualisation de la variation de signal (27) mémorisée dans l'autre mémoire (42), cette variation est remplacée par la variation dans le temps, mémorisée temporairement, du potentiel électrique du tissu.

3. Dispositif suivant la revendication 1, caractérisé par le fait qu'à la première mémoire (37) et à l'autre mémoire (42) est associé un dispositif de formation de la valeur moyenne (50), dans lequel pour l'actualisation de la variation de signal mémorisée dans l'autre mémoire (42), cette variation de signal et la variation dans le temps, mémorisée temporairement dans la première mémoire (37), du potentiel de tissu sont combinées pour fournir un signal de valeur moyenne, qui est mémorisé dans l'autre mémoire (42).

4.  Dispositif suivant l'une des revendications précédentes, caractérisé par le fait que le dispositif d'exploitation comporte un détecteur à valeur de seuil (59) servant à contrôler le signal de différence (29) pour déterminer s'il existe des dépassements d'une valeur de seuil prédéterminée S1.

5.  Dispositif suivant l'une des revendications 1 à 3, caractérisé par le fait que le dispositif d'exploitation (49) contient un intégrateur (71) pour intégrer le signal de différence (29) et un détecteur à valeur de seuil (59) branché en aval et servant à contrôler le signal de différence intégré (72) pour déterminer s'il dépasse une valeur de seuil prédéterminée S2.

6.  Dispositif suivant la revendication 4 ou 5, caractérisé par le fait que le détecteur à valeur de seuil (59) est une mémoire de valeur maximale (65) servant à détecter la valeur crête-à-crête SS1 ou SS2 du signal de différence (29) ou du signal de différence intégré (72).

7.  Dispositif suivant l'une des revendications précédentes, caractérisé par le fait que le générateur d'impulsions de stimulation (30) produit des impulsions de stimulation biphasées (26).

FIG 1

FIG 2

FIG 3

FIG 4

FIG 5

FIG 6

FIG 7

FIG 8